# EUROPEAN PATENT APPLICATION

(11) **EP 2 255 776 A1**
(43) Date of publication of application: **01.12.2010**
(21) Application number: 09161072.5
(22) Date of filing: 26.05.2009
(51) Int. Cl.: A61K 8/19, A61K 8/365, A61Q 5/02, A61Q 5/12, C11D 3/20

(54) **Cleansing composition**

(71) Applicant: PURAC Biochem BV, 4206 AC Gorinchem (NL)
(72) Inventor: Gijssen, Rupertus Mattias Rafaël, 4062 PP, Zennewijnen (NL); Yap, Kwee Chwee, 117640, Singapore (SG)
(74) Representative: Hesselink, Dinah Elisabeth

(57) **Abstract**

The present invention pertains to a cleansing composition comprising 4-55 wt.% of a surfactant, 0.1-15 wit.% of a combination of a lactate salt and a gluconate salt, wherein the lactate salt makes up 10-90 wt.% of the total of the lactate salt and the gluconate salt, and water.

It was found that the use of a combination of a lactate salt and a gluconate salt in a cleansing composition may serve to improve one or more of the mildness, the foaming properties, the skin feel properties, the viscosity properties, the skin hydration properties, the wet and dry combing properties, the anti-static properties, the hair softness and hair smoothness properties, the hair volume properties, and the hair bounce and hair lightness properties of a cleansing composition.

## Description

The present invention pertains to a cleansing composition and to a method for manufacturing a cleansing composition.

Within the context of the present specification a cleansing composition is a liquid composition which is suitable for cleansing a substrate in combination with water, and which is subsequently rinsed off. They may also be indicated as rinse-off compositions, to distinguish from leave-on compositions like moisturising creams and lotions. Examples of rinse-off cleansing compositions include compositions aimed at cleaning human or animal hair or body, such as shampoos, liquid soaps, body conditioners, shower gels, and foam baths. Cleansing compositions also include compositions aimed at cleansing other substrates, but which come into contact with the human body during the performance of the cleansing. Examples include manual dishwashing compositions, hard surface cleaning compositions, and manual laundry detergents.

Cleansing compositions have to meet a number of different requirements. In the first place, they have to show good cleansing properties of the substrate, whether it is hair, body, or objects such as tableware. Further, they should have good foaming properties. Their viscosity has to be such that the composition is neither too thick or too thin, and the composition should have a substantial stability over time. Depending on the application other properties may be important. A further property is mildness, that is, that the composition does not result in irritation of for example skin or eyes, or dryness, roughness or redness of the skin. A further property that may be important is skin feel, which pertains to the sensation associated with the use of the product. It includes the experience of the foaming properties, skin adherence, stickiness, ease-to-rinse, etc. For compositions that are to be applied onto the hair, important requirements include conditioning properties properties like improved combing properties, prevention of fly-way hair, improved gloss and volume, anti-static properties, and improved hair feel.

Generally, cleansing compositions comprise a primary surfactant to effect cleansing and foaming, and if so desired one or more secondary surfactants and further components like thickening agents to create the appropriate viscosity and stability, colouring or pearlizing agents, preservatives and perfumes. For hair-care products the optional presence of conditioning agents, lubricants, glossers and hair reconstructors may be mentioned.

There is a continuous need for the provision of improved cleansing compositions. In particular there is need for a cleansing composition which has an appropriate viscosity, which is mild in use, and shows good skin feel. Further, the composition should have good foam-forming properties with the foam having high stability. Additionally, depending on the composition, properties like improved combing properties, prevention of fly-way hair, improved gloss and volume, anti-static properties are important.

Further, a general trend in the field of cleansing compositions is to aim for a reduced surfactant concentration. Further, there is a desire to use compounds that may be obtained starting from renewable resources, through relatively simple processes.

The present invention provides a cleansing composition which solves the above problems. The cleansing composition according to the invention comprises 4-55 wt.% of a surfactant, 0.1-15 wt.% of a combination of a lactate salt and a gluconate salt, wherein the lactate salt makes up 10-90 wt.% of the total of the lactate salt and the gluconate salt, and water.

For good order's sake it is noted that, unless indicated otherwise, all wt.% are calculated as dry weight of active component. For the lactate salt and gluconate salt the weight% are calculated as lactate and gluconate anion, respectively.

It has been found that a cleansing composition comprising 0.1-15 wt.% of a combination of a lactate salt and a gluconate salt as stipulated above makes for a composition with a number of desirable properties, including good skin feel, good foam forming properties, and increased foam stability, increased skin hydration has also been observed, which is an indication of good mildness properties. Where the composition is used on hair, it was found that the combing properties of the hair, its softness and smoothness, and its volume were improved, while it suffered less from static build up or fly-away hair.

Further, the combination of a lactate salt and a gluconate salt was found suitable to replace the secondary surfactants using in cleaning compositions in whole or in part. This results in an overall reduction of surfactant level in the composition. More specifically, it has been found that the combination of a lactate salt and a gluconate salt according to the invention can, in whole or in part, replace cocamidopropyl betaine (CAPB), which is used as secondary surfactant in a large percentage of cleansing compositions. This is attractive because, as is known in the art, impurities that may be present in some grades of cocamidopropyl betaine may have skin sensitising properties, which may contribute to triggering allergic reactions. Accordingly, in one embodiment, the composition according to the invention contains less than 1 wt.% of cocamidopropyl betaine, in particular less than 0.7 wt.% of cocamidopropyl betaine, more in particular less than 0.3 wt.% of cocamidopropyl betaine, calculated on the total of the composition. If so desired, the composition can be free of cocamidopropyl betaine.

It has been found in clinical experiments that the use of the combination of a lactate salt and a gluconate salt gives the same mildness as a comparable composition comprising cocamidopropyl betaine.

It is noted that the use of lactate and gluconate salts individually in cleansing compositions is known in the art. US 5,380,756 describes the use of sodium lactate in shampoo as moisturiser.

US 2004/0234489 describes the use of sodium gluconate in hair conditioner for softening.

US 5,378,731 describes a shampoo comprising lactic acid as chelating agent, and sodium lactate as moisturiser.

US 6,087,320 describes sodium lactate and gluconate as viscosity improver in a surfactant concentrate.

WO 2007/068399 describes the sodium salt of lactic acid in hair-care compositions.

However, none of the references cited above describe the use of 0.1-15 wt.% of a combination of a lactate salt and a gluconate salt, wherein the lactate salt makes up 10-90 wt.% of the total of the lactate salt and the gluconate salt. It has been found that the combined use of a lactate salt and a gluconate salt shows a number of synergistic effects as compared with the use of either compound alone. More in particular, looking at viscosity, experiments have shown that the use of sodium gluconate alone results in a composition with a viscosity which is too low. On the other hand, the use of sodium lactate alone results in a composition with a viscosity which is much too high. The calculated mean value of the two is still too high. However, surprisingly it has been found that the use of a combination of the two components results in a viscosity which is appropriate for use in rinse-off cleansing compositions. As to foam forming and foam stability, sodium lactate and the combination of sodium lactate and sodium gluconate were found to score equal for fresh foam, and better than the use of sodium gluconate alone. However, the stability of the foam of a composition comprising the combination of sodium lactate and sodium gluconate is better than the stability of comparable compositions comprising either sodium lactate alone or sodium gluconate alone. Further, a composition comprising either of the two components by itself scored substantially less on the skin-feel test than a comparable composition comprising the combination of the two components. The same goes for the skin hydration.

In the present invention, a combination is used of a lactate salt and a gluconate salt. The lactate salt and the gluconate salt may be selected from the lactate and gluconate salts known in the art for cleansing applications. For example, the lactate salt may be selected from one or more of sodium lactate, potassium lactate, ammonium lactate, calcium lactate, and triethanolamine lactate. For example, the gluconate salt may be selected from one or more of sodium gluconate, potassium gluconate, ammonium gluconate, calcium gluconate, and triethanolamine gluconate.

In general, the use of ammonium salts may be less preferred because these compositions necessitate careful processing to prevent the formation of ammonia. Calcium salts may be attractive for their hydration properties, but they may interfere with the surfactant. Therefore, it may be preferred for the lactate salt to consists for at least 50 wt.% of sodium and/or potassium lactate, in particular for at least 70 wt.%, more in particular for at least 90%, still more in particular for at least 95%.

Along the same lines, it may be preferred for the gluconate salt to consists for at least 50 wt.% of sodium and/or potassium gluconate, in particular for at least 70 wt.%, more in particular for at least 90%, still more in particular for at least 95%.

The use of sodium lactate and sodium gluconate is considered particularly preferred. Therefore, it may be preferred for the lactate salt to consists for at least 50 wt.% of sodium lactate, in particular for at least 70 wt.%, more in particular for at least 90%, still more in particular for at least 95%, and/or for the gluconate salt to consist for at least 50 wt.% of sodium gluconate, in particular for at least 70 wt.%, more in particular for at least 90%, still more in particular for at least 95%.

As indicated above, the composition according to the invention comprises 0.1-15 wt.% of the specified combination of a lactate salt and a gluconate salt. If the amount of the combination is too low, the effect of the present invention will not be obtained. It may be preferred for the combination of lactate salt and gluconate salt to be present in an amount of at least 0.3 wt.%, in particular at least 0.5 wt.%. On the other hand, the use of more than 15 wt.% will generally not be associated with additional advantages. It may be preferred for the cleansing composition to comprise the combination of a lactate salt and a gluconate salt in an amount of up to 10 wt.%, more in particular up to 5 wt.%, still more in particular up to 3 wt.%.

It is essential to the present invention that the cleansing composition comprise both a lactate salt and a gluconate salt. The lactate salt makes up 10-90 wt.% of the total of lactate salt and gluconate salt. More in particular, it may be preferred for the combination of lactate salt and gluconate salt to comprise between 20 and 80 wt.% of lactate salt, more in particular between 30 and 70 wt.% of lactate salt, still more in particular between 40 and 60 wt.% of lactate salt, the balance being gluconate salt.

In one embodiment of the present invention, the surfactant concentration is relatively high. This may be for example, in the case of compositions suitable for use in dishwashing or other cleaning applications. In this embodiment, the surfactant concentration is for example in the range of 15-55 wt.%.

In another embodiment of the present invention, the surfactant concentration is relatively low, for example in the range of 4-30 wt.% This is for example the case for compositions suitable for cleansing hair or body, and for the lower concentrated dishwashing compositions.

Suitable surfactants for use in cleansing compositions are known in the art. They comprise, e.g. anionic surfactants like sulphate-based surfactants and sulphonate-based surfactants. Examples of suitable surfactants include laureth sulphates, in particular sodium or ammonium laureth sulphate, lauryl sulphates, in particular sodium lauryl sulphate, cumene sulphonates, in particular sodium cumene sulphonate, xylene sulphonates, in particular sodium xylene sulphonate, and alkane sulphonates, in particular sodium alkane sulphonate.

In another embodiment of the present invention, relatively mild surfactants such as sodiumcocoyl isethionate, and sarcosinates may be used. However, it is believed that the advantages of the present invention will be more pronounced when sulphate-based surfactants or sulphonate-based surfactants are used.

In particular for compositions that are to be used for cleansing hair, the composition may also comprise cationic polymers, such as polyquaternium polymers and cationic guar, as these may have a positive effect on the combing properties of the hair. Incidentally, it has been found that the composition according to the invention gives good hair-care properties also in the absence of these cationic polymers. Accordingly, the present invention also pertains to a compositions suitable for the cleansing of hair which contains less than 1 wt.% of cationic polymers, in particular less than 0.5 wt.%, still more in particular less than 0.2 wt.%, even more in particular less than 0.1 wt.%, or even less than 0.05 wt.%. In one embodiment, the composition is free from cationic polymers.

Other components which may suitably be included in compositions according to the invention include secondary surfactants, such as cocamidopropyl betaine, disodiumlaureth sulphosuccinate, cyclotriazadisulphonamide, cocomethylethylamide, cocodimethylethylamide, PEG-5 laurylcitrate sulphosuccinate, monosodiumcocamphoacetate, and disodiumcocamphodiacetate.

The composition may additionally comprise one or more additional components known in the art for cleansing compositions.

For example, if so desired, the composition may comprises a thickening agent and/or rheology modifyer such as cocomethylethylamide, cocodimethylethylamide, and acrylate copolymers, e.g., in an amount of 0.1- 3 wt.%. If so desired, the composition may also comprise a pH regulating agent, e.g., in an amount of 0.1-3 wt.%. The composition may also comprise one or more compounds selected from the group of preservatives and antibacterial agents, e.g., in an amount of 0.1-3 wt.%, frangrance compounds, e.g., in an amount of 0.1-1 wt.%, and colouring or pearlescent agents, e.g., in an amount of 0.1-1 wt.%. Where applicable, the composition may contain conditioning agents, e.g., in an amount of 0.5-7 wt.%, hydrolysed proteins as hair reconstructors, e.g., in an amount of 0.1-0.5 wt.%, and conditioners and emollients such as glycerine, e.g., in an amount of 2-7 wt.%. These compounds are known in the art and require no further elucidation here.

The composition generally has a viscosity (dynamic, spindle LV3, 10 rpm, 20°C) in the range of 100 to 40000 mPa.s. For dishwashing compositions a range of 200-1000 mPa.s may be mentioned. For compositions for cleansing hair a range of 4000 to 10000 mPa.s may be mentioned. For bodywash cleansing compositions a range of 20000 to 32000 mPa.s may be mentioned. For hand wash a range of 5000 to 15000 mPa.s may be mentioned.

The composition generally has a pH in the range of 4.5 to 7.5, more in particular in the range of 5 to 7, still more in particular in the range of 5.5 to 6.5.

The present invention also pertains to a process for manufacturing the cleansing composition described above, which comprises the steps of combining and homogenising 4-55 wt.% of a surfactant, 0.1-15 wt.% of the combination of lactate salt and gluconate salt and water. The components may be provided in any form, depending on their nature, e.g., in the form of solid composition, dispersions or solutions in water or other solvents, pastes, or in any other form.

In one embodiment, in a first step surfactants are combined with water until a homogeneous solution, then the other components are added, in any desirable form, then, the pH and the viscosity are adjusted to the desired value. In general, the pH of the composition is adjusted first, followed by the adjustment of the viscosity.

The lactate and gluconate salts may be combined with the other ingredients in separate steps. They may also be combined with the other ingredients in a single step. In one embodiment in a first step a mixture is prepared which comprises the lactate and gluconate salts, and in a second step the mixture is combined with the other components. This mixture may, for example, be in the form of an aqueous solution, e.g., a solution with a concentration of at least 10 wt.%. The upper limit of the concentration will be determined by solubility considerations and will generally be of the order of 38 wt.%. It may be preferred for the concentration of the solution to be at least 20 wt.%, in particular at least 30 wt.%, as more concentrated, solutions make for more efficient processing.

It may be attractive for the mixture of lactate and gluconate salts which is prepared in the first step to additionally comprise 1-10 wt.% of NaCl, calculated on the total of the mixture, to help in adjusting the viscosity of the final composition. The mixture of lactate and gluconate salts may also comprise further components such as preservatives.

The present invention also pertains to a process for improving one or more of the mildness, the foaming properties, the skin feel properties, the viscosity properties, the skin hydration properties, the wet and dry combing properties, the anti-static properties, the hair softness and hair smoothness properties, the hair volume properties, and the hair bounce and hair lightness properties of a cleansing composition, by incorporating therein 0.1-15 wt.% of a combination of a lactate salt and a gluconate salt as described above, wherein the lactate salt makes up 10-90 wt.% of the total of the lactate salt and the gluconate salt is incorporated into the composition.

The present invention also pertains to the use for improving one or more of the mildness, the foaming properties, the skin feel properties, the viscosity properties, the skin hydration properties, the wet and dry combing properties, the anti-static properties, the hair softness and hair smoothness properties, the hair volume properties, and the hair bounce and hair lightness properties of a cleansing composition of a combination of a lactate salt and a gluconate salt, wherein the lactate salt makes up 10-90 wt.% of the total of the lactate salt and the gluconate salt.

For preferred embodiments, reference is made to what has been stated above for the composition.

The present invention will be elucidated by the following examples, without being limited thereto or thereby.

### Example 1

Compositions were prepared comprising 14 wt.% of sodium laureth sulphate, 4 wt.% of cocamide monoethanolamine, 0,875 wt.% of active ingredient, and the balance water. The compositions were brought to a pH of 5, using lactic acid. The active ingredients used are listed in Table 1.

**Table 1**

| Composition | Active ingredient |
|---|---|
| A (comparative) | none |
| B (comparative) | cocamidopropyl betaine |
| C (comparative) | sodium gluconate |
| D (comparative) | sodium lactate |
| E | 50:50 sodium gluconate: sodium lactate |

The viscosity of the composition was determined as follows: 100 ml of formulation was put in a beaker glass (all the same size 100 ml glasses) The viscosity was measured with a Brookfield viscosity meter (model DV-III Reprogrammable Rheometer) with spindle nr 3 at 20°C. The outcome at a spinning of 10 rpm was noted in mPa.s.

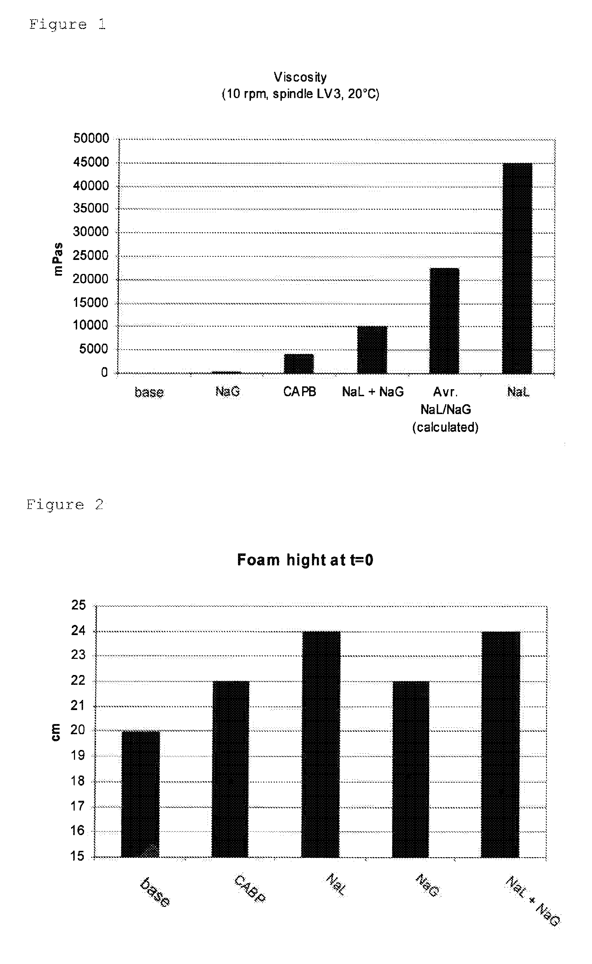

The results are presented in Figure 1. The viscosity aimed for is between 5000 to 15000 mPa.s for hand wash compositions. As can be seen from the figure, the use of sodium gluconate alone resulted in a composition with a viscosity which is too low. On the other hand, the use of sodium lactate alone resulted in a composition with a viscosity which is much too high. It is interesting to note that the calculated mean value of these two compositions is still too high. However, surprisingly it has been found that the use of a combination of sodium lactate and sodium gluconate resulted in a composition which after pH adjustment has a viscosity within the desired range.

Each formulation was diluted with water to a concentration of 3 wet.%. The stability of foam and the foam forming was determined by pouring 70 ml of 3% w/w solution of the formulation in a volumetric flask of 250 ml and shaking 10 times very hard. The foam layer was measured in cm immediately and after 5 minutes.

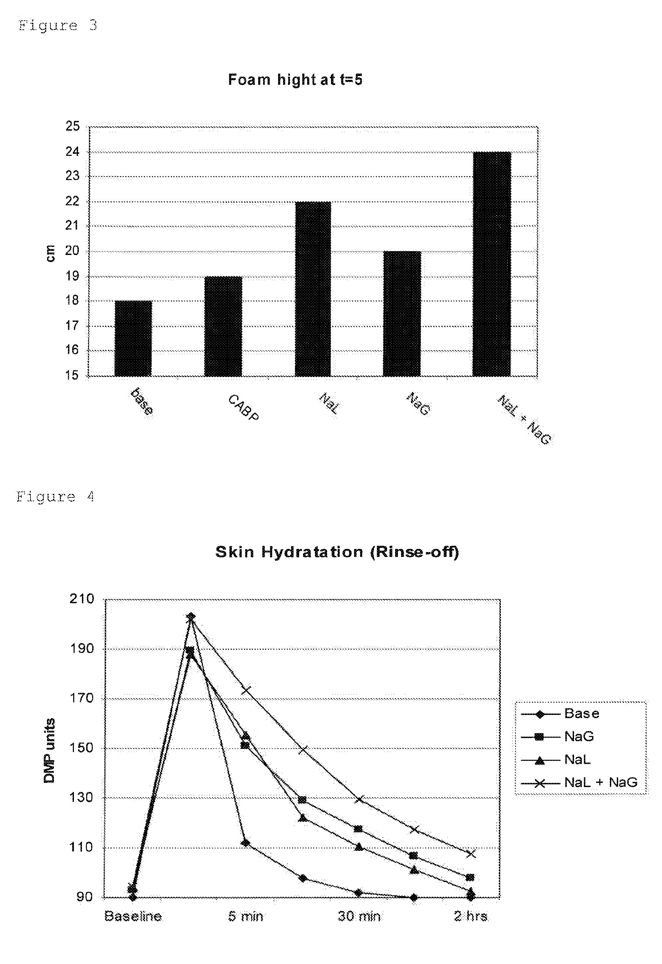

The results are presented in Figures 2 and 3. As can be seen from the figure, sodium lactate and the combination of sodium lactate and sodium gluconate score equal for fresh foam, and better than the use of sodium gluconate alone. However, the stability of the foam of a composition comprising the combination of sodium lactate and sodium gluconate is better than the stability of comparable compositions comprising either sodium lactate alone or sodium gluconate alone.

The formulations were also subjected to a skin-feel test by six test persons. The compositions were provided in numbered 10 ml cups, in random order. The test persons did not know which composition was in which cup. The test persons used the content of the cups wash their hands. The hands were rinsed, and dried with a towel. They were then allowed to dry for 30 minutes. The compositions were evaluated for viscosity, foaming, stickiness, ease-to-rinse, and skin feel after drying. Each test person graded the compositions with a figure from 1 to 7, wherein 1 referred to the worst composition and 7 referred to the best composition. The total ranking of the compositions is presented in table 2.

**Table 2**

| Composition | Active ingredient | Total ranking |
|---|---|---|
| A (comparative) | none | 6 |
| B (comparative) | cocamidopropyl betaine | 20 |
| C (comparative) | sodium gluconate | 27 |
| D (comparative) | sodium lactate | 12 |
| E | 50:50 sodium gluconate sodium lactate | 40 |

As can be seen from Table 2, composition E according to the invention scored much better that the comparative compositions.

### Example 2

To evaluate the moisturising effect of the combination of sodium lactate and sodium gluconate test compositions were prepared containing 7.5 wt.% of acyl glutamate, 2 wt.% of respectively, sodium lactate, sodium glutamate, or a 50:50 wt.% mixture of sodium lactate and sodium gluconate, distilled water, and sodium hydroxide in an amount sufficient to bring the pH to 7. It is noted that the composition was kept as simple as possible to avoid any potential disturbance by other ingredients.

The compositions were submitted to clinical testing as follows: Test subjects were selected between the ages 35-55. In the four days before testing, they were only allowed to use a standardised soap to wash their facial area. The test was carried out by applying 2 mg product per cm2 of skin and washing for 30 seconds with gentle rubbing and warm water (41-43°C). Moisture content of the center of the cheek area was determined using a Nova Impedance Meter. Prior to testing the test subjects equilibrated for 30 minutes at 30-40 RH and 20-22C.

The test results are given in Figure 4. As can be seen from the figure, the composition containing the combination of sodium lactate and sodium gluconate and the base case have an equal DMP value one minute after start. The compositions containing sodium lactate alone or sodium gluconate alone have a lower DMP value. The DMP value of all compositions decreases over time. As can be seen from the figure, the composition according to the invention has the highest DMP value over the entire tested time frame.

### Example 3

In this example, various shampoo variation of the composition according to the invention were evaluated and compared to compositions not according to the invention.

The composition of the various compositions is give in table 3 below. All compositions were prepared by a process comprising the steps of dissolving the sodium laureth sulphate in water, combining it with the other components followed by intense mixing, and adjusting it to the required pH value with lactic acid.

**table 3**

| compound (wt.%) | A (comp) | B (inv) | C (comp) | D (inv) | E (comp) | F (inv) |
|---|---|---|---|---|---|---|
| Sodium laureth sulphate (SLES) | 7 | 9.45 | 7 | 9.45 | 7 | 9.45 |
| cocamidopropyl betaine (CAPB) | 2.4 | - | 2.4 | - | 2.4 | - |
| 50:50 sodium glutamatate: sodium lactate (wt.%) | - | 1.4 | - | 1.4 | - | 1.4 |
| cocodiethanolamide | 3 | 3 | 3 | 3 | 3 | 3 |
| sodium chloride | 0.5 | 2 | 0.5 | 2 | 0.5 | 2 |
| dimethicone | - | - | - | - | 1 | 1 |
| polyquaternium 10 | - | - | 0.3 | - | 0.3 | - |
| glycerine | 0.5 | - | 0.5 | - | 0.5 | - |
| preservative | q.s. | q.s. | q.s. | q.s. | q.s. | q.s. |
| fragrance | q.s. | q.s. | q.s. | q.s. | q.s. | q.s. |
| lactic acid | to pH 5.5 | to pH 5.5 | to pH 6.0 | to pH 6,0 | to pH 6.0 | to pH 6.0 |
| demineralised water | to 100% | to 100% | to 100% | to 100% | to 100% | to 100% |

In the above table q.s. stands for quotes status, which means in the required amount.

All compositions were tested by washing the hair of a test doll. The comparative composition was used on one half of the hair, the composition according to the invention was used on the other half of the hair.

A comparison between composition A (comparative), and composition B (according to the invention showed the following:
Both compositions give a rich foam. For both compositions wet combing can be improved. Composition B gives a better performance than composition A in that it leaves the hair softer and more lustrous, in gives better manageability of the hair, it provides a smoother hair feel, it gives more body (volume) to the hair, and it is easier to comb, both wet and dry.

Composition C, which is the same as composition A except that it also contains polyquaternium 10 (PQ10), has an improved volume and easier wet and dry comb, as compared to Composition A. This same effect is observed in composition D according to the invention, which does not contain PQ10. Additionally, composition D gives a smoother hair feel than composition C.

A comparison between composition E, which is comparative, and composition F, which is according to the invention, shows that both formulations give rich foam, both formulations leave the hair soft, and both formulations provide good wet and dry comb. Additionally, composition F according to the invention results in hair with more volume, which feels light, with more bounce, and which is smoother. Further, the composition according to the invention results in hair with less static build-up, in other words, less fly-away hair.

## Claims

1. Cleansing composition comprising 4-55 wt.% of a surfactant, 0.1-15 wt.% of a combination of a lactate salt and a gluconate salt, wherein the lactate salt makes up 10-90 wt.% of the total of the lactate salt and the gluconate salt, and water.

2. Cleansing composition according to claim 1 wherein the lactate salt is selected from one or more of sodium lactate, potassium lactate, ammonium lactate, calcium lactate, and triethanolamine lactate and the gluconate salt is selected from one or more of sodium gluconate, potassium gluconate ammonium gluconate, calcium gluconate, and triethanolamine gluconate.

3. Cleansing composition according to claim 1 or 2 wherein the wherein the lactate salt consists for at least 50 wt.% of sodium and/or potassium lactate, in particular for at least 70 wt.%, more in particular for at least 90%, still more in particular for at least 95%, and/or the gluconate salt consists for at least 50 wt.% of sodium and/or potassium gluconate, in particular for at least 70 wt.%, more in particular for at least 90%, still more in particular for at least 95%.

4. Cleansing composition according to claim 3, wherein the lactate salt consists for at least 50 wt.% of sodium lactate, in particular for at least 70 wt.%, more in particular for at least 90%, still more in particular for at least 95%, and/or the gluconate salt consists for at least 50 wt.% of sodium gluconate, in particular for at least 70 wt.%, more in particular for at least 90%, still more in particular for at least 95%.

5. Cleansing composition according to any one of the preceding claims, wherein the combination of lactate and gluconate salts comprises between 20 and 80 wt.% of lactate salt, more in particular between 30 and 70 wt.% of lactate salt, still more in particular between 40 and 60 wt.% of lactate salt, the balance being gluconate salt.

6. Cleansing composition according to any one of the preceding claims, wherein the combination of lactate salt and gluconate salt is present in an amount of at least 0.3 wt.%, in particular at least 0.5 wt.%, the amount being up to 10 wt.%, more in particular up to 5 wt.%, still more in particular up to 3 wt.%.

7. Cleansing composition according to any one of the preceding claims wherein the amount of surfactant is in the range of 15-55 wt.%.

8. Cleansing composition according to any one of the preceding claims 1-6, wherein the amount of surfactant is in the range of 4-30 wt.%.

9. Cleansing composition according to any one of the preceding claims, which contains less than 1 wt.% of cocamidopropyl betain, in particular less than 0.7 wt.% of cocamidopropyl betain, more particular less than 0.3 wt.% of cocamidopropyl betaine, calculated on the total of the composition.

10. Process for manufacturing a cleansing composition according to any one of the preceding claims which comprises the step of combining and homogenising: 4-55 wt.% of a surfactant, 0.1-15 wt.% of a combination of a lactate salt and a gluconate salt, wherein the lactate salt makes up 10-90 wt.% of the total of the lactate salt and the gluconate salt, and water.

11. Process according to claim 10, wherein the lactate salt and the gluconate salt are combined with the other components in separate steps.

12. Process according to claim 10, wherein in a first step a mixture is prepared which comprises a lactate salt and a gluconate salt, and in a second step the mixture is combined with the other components.

13. Process according to claim 12, wherein the mixture comprising a lactate salt and a gluconate salt is in the form of an aqueous solution, in particular in the form of a solution with a concentration of at least 10 wt.%, more in particular of at least 20 wt.%, still more in particular of at least 30 wt.%.

14. Process for improving one or more of the mildness, the foaming properties, the skin feel properties, the viscosity properties, the skin hydration properties, the wet and dry combing properties, the anti-static properties, the hair softness and hair smoothness properties, the hair volume properties, and the hair bounce and hair lightness properties of a cleansing composition wherein 0.1-15 wt.% of a combination of a lactate salt and a gluconate salt, wherein the lactate salt makes up 10-90 wt.% of the total of the lactate salt and the gluconate salt is incorporated into the composition.

15. Use for improving one or more of the mildness, the foaming properties, the skin feel properties, the viscosity properties, the skin hydration properties, the wet and dry combing properties, the anti-static properties, the hair softness and hair smoothness properties, the hair volume properties, and the hair bounce and hair lightness properties of a cleansing composition of a combination of a lactate salt and a gluconate salt, wherein the lactate salt makes up 10-90 wt.% of the total of the lactate salt and the gluconate salt.
